Europäisches Patentamt

European Patent Office

Office européen des brevets

(11) Publication number: **0 354 970 B1**

# EUROPEAN PATENT SPECIFICATION

(45) Date of publication of patent specification: **04.05.94** (51) Int. Cl.⁵: **C07C 69/96**, C07C 68/00

(21) Application number: **89902082.0**

(22) Date of filing: **07.02.89**

(86) International application number:
**PCT/JP89/00122**

(87) International publication number:
**WO 89/07587 (24.08.89 89/20)**

The file contains technical information submitted
after the application was filed and not included in
this specification

(54) **PROCESS FOR PRODUCING CARBONIC ACID ESTER.**

(30) Priority: **16.02.88 JP 33572/88**
**04.01.89 JP 202/89**

(43) Date of publication of application:
**21.02.90 Bulletin 90/08**

(45) Publication of the grant of the patent:
**04.05.94 Bulletin 94/18**

(84) Designated Contracting States:
**DE FR IT**

(56) References cited:
**EP-A- 0 090 977**
**WO-A-87/07601**
**JP-A- 5 424 827**
**JP-A-62 167 483**

(73) Proprietor: **DAICEL CHEMICAL INDUSTRIES, LTD.**
**No. 1-Banchi, Teppo-cho**
**Sakai-shi Osaka-fu 590(JP)**

(72) Inventor: **YOKOTA, Shigeru**
**500, Kamiyobe, Yobe-ku**
**Himeji-shi Hyogo 671-12(JP)**
Inventor: **TANAKA, Yasutaka**
**500, Kamiyobe, Yobe-ku**
**Himeji-shi Hyogo 671-12(JP)**
Inventor: **MIYAKE, Hiroto**
**Shinzaike, Aboshi-ku**
**Himeje-shi Hyogo 671-12(JP)**

(74) Representative: **Patentanwälte Grünecker,**
**Kinkeldey, Stockmair & Partner**
**Maximilianstrasse 58**
**D-80538 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The invention relates to a process for producing a carbonic acid ester or carbonic ester by reacting an alcohol with carbon monoxide and oxygen with a catalyst. The carbonic esters are industrially quite important compounds usable as intermediates in the production of polymers, medicines and pesticides and also as solvents.

Proposed processes for the production of carbonic esters include, for example, a process wherein an alcohol is reacted with carbon monoxide and oxygen in the presence of a catalyst which comprises a cuprous halide and an alkali metal halide or alkaline earth metal halide (cf. Japanese Patent Publication No. 8020/1981) and a process wherein an alcohol is reacted with carbon monoxide and oxygen in the presence of a copper compound or a combination of a copper compound with, e.g., a platinum group compound and alkali metal salt, (cf. Japanese Patent Publication No. 8816/1986).

However, the former process has the problem that the velocity of forming the carbonic ester is extremely low and the latter process has the problem that an erosion of the equipment and piping of the plant occurs due to a poor solubility of the catalyst components.

WO-A-87/07601 describes a process for the preparation of dihydrocarbyl carbonates employing a heterogeneous catalyst which comprises a metal halide or mixed metal halide impregnated on a support, wherein the mixed metal halide consists of cupric chloride and magnesium, cesium or calcium chloride. This preparation is carried out in the vapour phase.

EP-A-90977 relates to a process for the production of alkyl, cycloalkyl or aralkyl carbonates using a catalyst containing a cobalt salt, a palladium salt and an alkali metal carboxylate (potassium acetate).

It is the object of the present invention to provide a process for producing a carbonic ester in an advantageous manner by increasing the velocity of forming it while using an as homogeneous as possible catalyst.

According to the present invention this object is attained with a process for producing a carbonic acid ester by reacting an alcohol with carbon monoxide and oxygen in the presence of a catalyst homogeneously dissolved in the reaction solution, said catalyst comprising a cupric salt and an alkaline earth metal salt, the cupric salt being a weak acid salt and/or a halide and the alkaline earth metal salt being a weak acid salt and/or a halide.

The inventors have found that when the catalyst according to the invention comprising a cupric salt and an alkaline earth metal salt is used in the production of a carbonic ester, a high solubility of copper can be obtained, the catalyst is homogeneously dissolved in the reaction solution and the formation velocity is increased.

The catalyst comprises, for example, a combination of (a) a cupric salt of a weak acid with (b) an alkaline earth metal halide or a combination of (a) a cupric salt of a weak acid and a platinum group compound with (b) an alkaline earth metal halide.

Among the catalysts used in the present invention, the cupric salts include, for example, cupric carboxylates such as cupric acetate, pivalate and benzoate; cupric salts of weak acids such as hydrobromic acid, carbonic acid and phenols, e.g. phenol, cresol and p-chlorophenol; and cupric halides such as cupric chloride and bromide. The amount of the cupric salt used is 1 to 3,000 mmol, preferably 10 to 1,000 mmol, per liter of the alcohol.

The alkaline earth metal compounds usable together with the cupric salt include, for example, chlorides, iodides and acetates of beryllium, magnesium, calcium and barium. The amount of the alkaline earth metal compound is preferably 1/10 to 10 mol per mol of the cupric salt. It is particularly preferred that the atomic ratio of the halogen to copper in the catalyst system is higher than 1/2 and lower than 2.

The platinum group compound used as the catalyst component includes, for example, a halide, acetate and nitrate of ruthenium, rhodium or palladium. Among them, the palladium salt is preferred. The amount of the platinum group compound used is at most 1 mol, preferably at most 1/10 mol, per mole of the cupric salt.

The alcohols used as the reactant in the present invention include, for example, saturated aliphatic alcohols such as methanol and ethanol; unsaturated aliphatic alcohols such as allyl alcohol; aromatic alcohols such as phenol; diols; and polyols. Among them, alcohols having 1 to 20 carbon atom are preferred and methanol is particularly preferred.

Carbon monoxide and oxygen which are gaseous reactants are not limited to those having a high purity but those diluted with an inert gas such as nitrogen, argon or carbon dioxide are usable. Therefore, air can be used as the oxygen source.

The reaction of the present invention is conducted in the presence of the above-described catalyst under atmospheric or elevated pressure, preferably at 98 to $98 \times 10^2$ kPa (1 to 100 atm). When the gas inert to the reaction is used for the dilution, the partial pressures of carbon monoxide and oxygen are adjusted in the ranges of 9.8 to $29 \times 10^2$ kPa (0.1 to 30 atm) and 4.9 to 980 kPa (0.05 to 10 atm), respectively. The reaction temperature ranges from 20 to 250°C. This reaction can be conducted either batchwise or continuously.

It will be understood from the above description that according to the process of the present invention, the velocity of forming the carbonic ester by reacting an alcohol with carbon monoxide and oxygen is increased in the presence of the catalyst comprising the cupric salt and the selectivity toward the intended product is increased as compared with those obtained when an ordinary catalyst comprising a cuprous halide is used. Therefore, when the process of the present invention is employed for the production of a carbonic ester, the production time can be reduced and the yield of the product can be remarkably increased. Another great advantage of the present invention is that the erosion of the equipment and piping of the plant can be controlled, since the catalyst is homogeneously dissolved in the reaction solution.

(Examples)

The following Examples will further illustrate the present invention.

Example 1

DMC (dimethyl carbonate) was produced in a 380 mℓ autoclave coated with Teflon. 40 mℓ of a solution of 7.5 mmol/ℓ of palladium chloride, 187.5 mmol/ℓ of cupric acetate and 187.5 mmol/ℓ of magnesium chloride in methanol were used as the catalyst. The catalyst was placed in the autoclave and then 12.0 kg/cm$^2$ of a gaseous mixture comprising 47.5 vol. % of nitrogen, 30 vol. % of carbon monoxide and 22.5 vol. % of argon/oxygen (oxygen content: 33.0 vol. %) were introduced thereinto. The temperature in the autoclave was elevated to 130°C and the reaction was conducted for 1 h. After the completion of the reaction, the autoclave was cooled and the amounts of carbon monoxide, oxygen, carbon dioxide and formed DMC were determined according to gas chromatography. The results are shown in Table 1.

Comparative Example 1

The reaction was conducted in the same manner as that of Example 1 except that cupric acetate used as the catalyst was replaced with cuprous chloride. The products were determined according to gas chromatography. The results are shown in Table 1.

Example 2

The reaction was conducted in the same manner as that of Example 1 except that 187.5 mmol/ℓ of cupric acetate and 187.5 mmol/ℓ of magnesium chloride were used as the catalyst. The products were determined to obtain the results shown in Table 1.

Comparative Example 2

The reaction was conducted in the same manner as that of Example 2 except that cupric acetate used as the catalyst was replaced with cuprous chloride. The results are shown in Table 1.

3

Table 1

| | Catalyst composition (mmol/ℓ) | | | | Amount of formed DMC (mmol) | Selectivity toward DMC S(CO₂-DMC) (%) |
|---|---|---|---|---|---|---|
| | $PdCl_2$ | $Cu(OAc)_2$ | $CuCl$ | $MgCl_2$ | | |
| Example 1 | 7.5 | 187.5 | | 187.5 | 9.9 | 32 |
| Comp. Ex. 1 | 7.5 | | 187.5 | 187.5 | 4.9 | 14 |
| Example 2 | | 187.5 | | 187.5 | 5.2 | 21 |
| Comp. Ex. 2 | | | 187.5 | 187.5 | 3.7 | 14 |

Selectivity toward DMC:

$$S(CO_2\text{-}DMC) = \frac{(\text{Amount of formed DMC})}{(\text{Amount of formed DMC}) + (\text{Amount of formed } CO_2)} \times 100$$

It is apparent from the results shown in Table 1 that when cupric acetate was used in place of cuprous chloride, the velocity of forming the carbonic ester was increased and the selectivity toward the carbonic ester was also increased and that when the palladium compound was used together with the copper salt, the amount of formed DMC and the selectivity were increased.

Example 3

The reaction was conducted in the same manner as that of Example 1 except that 19.4 kg/cm$^2$ of a gaseous mixture comprising 63.0 vol. % of nitrogen, 27.8 vol. % of carbon monoxide and 27.8 vol. % of argon/oxygen (oxygen content: 33.0 vol. %) were used. The products were determined according to gas chromatography. The results are shown in Table 2. The solubility of Cu determined after the completion of the reaction is also shown therein.

Comparative Example 3

The reaction was conducted in the same manner as that of Example 3 except that magnesium chloride used as the catalyst was replaced with sodium chloride. The results are shown in Table 2.

Comparative Example 4

The reaction was conducted in the same manner as that of Example 3 except that magnesium chloride used as the catalyst was replaced with potassium chloride. The results are shown in Table 2.

Table 2

| | Catalyst composition (mmol/ℓ) | | | | | Amount of formed DMC (mmol) | Selectivity toward DMC S($CO_2$-DMC) (%) | Solubility of Cu (%) |
|---|---|---|---|---|---|---|---|---|
| | $PdCl_2$ | Cu(IIAc)$_2$ | $MgCl_2$ | NaCl | KCl | | | |
| Example 3 | 7.5 | 187.5 | 187.5 | | 9.9 | 13.0 | 39.0 | 100 |
| Comp. Ex. 3 | 7.5 | 187.5 | | 187.5 | | 15.2 | 46.6 | 39 |
| Comp. Ex. 4 | 7.5 | 187.5 | | | 187.5 | 8.9 | 26.5 | 28 |

6

Selectivity toward DMC:

$$S(CO_2\text{-}DMC) = \frac{(\text{Amount of formed DMC})}{(\text{Amount of formed DMC}) + (\text{Amount of formed } CO_2)} \times 100$$

It is apparent from the results shown in Table 2 that in Example 3 wherein the reaction pressure was higher than that of Example 1, the solubility of copper in the reaction solution was higher, the amount of formed DMC was larger and the selectivity was higher than that of Example 3. It is also apparent that when magnesium chloride was replaced with sodium chloride or potassium chloride, the catalyst system was heterogeneous due to the poor solubility of the catalyst to erode the equipment and piping of the plant. Namely, in Example 3, the solubility of copper was higher than that of Comparative Example 3 or 4 and it is homogeneously dissolved in the reaction solution and, therefore, the erosion of the equipment and piping of the plant can be controlled.

Example 4

38 mℓ of a 40 wt. % solution of DMC in methanol and a catalyst system comprising 0.17 mmol/ℓ of palladium chloride, 19 mmol/ℓ of copper acetate, 12.7 mmol/ℓ of magnesium acetate and 6.3 mmol/ℓ of magnesium chloride were placed in a 320- mℓ autoclave lined with glass. Then 12 kg/cm² of a gaseous mixture comprising 77.5 vol. % of nitrogen, 15 vol. % of carbon monoxide and 7.5 vol. % of oxygen were introduced thereinto. The temperature in the autoclave was elevated to 130°C and the reaction was conducted for 1 h. After the completion of the reaction, the autoclave was cooled and the amounts of carbon monoxide, oxygen, carbon dioxide and formed DMC were determined according to gas chromatography. The results are shown in Table 3.

Example 5

The reaction was conducted in the same manner as that of Example 4 except that 0.17 mmol/ℓ of palladium chloride, 4.7 mmol/ℓ of cupric acetate, 14.3 mmol/ℓ of cupric chloride and 19 mmol/ℓ of magnesium acetate were used as the catalyst components. The products were determined according to gas chromatography. The results are shown in Table 3.

Example 6

The reaction was conducted in the same manner as that of Example 4 except that 0.17 mmol/ℓ of palladium chloride, 6.3 mmol/ℓ of cupric acetate, 12.7 mmol/ℓ of cupric chloride and 19 mmol/ℓ of magnesium acetate were used as the catalyst components. The products were determined according to gas chromatography. The results are shown in Table 3.

Example 7

The reaction was conducted in the same manner as that of Example 4 except that 0.17 mmol/ℓ of palladium chloride, 9.5 mmol/ℓ of copper acetate, 9.5 mmol/ℓ of cupric chloride and 19 mmol/ℓ of magnesium acetate were used as the catalyst components. The products were determined according to gas chromatography. The results are shown in Table 3.

Example 8

The reaction was conducted in the same manner as that of Example 4 except that 0.17 mmol/ℓ of palladium chloride, 12.7 mmol/ℓ of copper acetate, 6.3 mmol/ℓ of cupric chloride and 19 mmol/ℓ of magnesium acetate were used as the catalyst components. The products were determined according to gas chromatography. The results are shown in Table 3.

Example 9

The reaction was conducted in the same manner as that of Example 4 except that 0.17 mmol/ℓ of palladium chloride, 19 mmol/ℓ of copper acetate and 19 mmol/ℓ of magnesium chloride were used as the catalyst components. The products were determined according to gas chromatography. The results are shown in Table 3.

**Table 3**

| | Catalyst composition (mmol/$\ell$) | | | | | Activity (mol/$\ell \cdot$ h) | | Selectivity (%) $S(CO_2\text{-}DMC)$ |
|---|---|---|---|---|---|---|---|---|
| | $PdCl_2$ | $Cu(OAc)_2$ | $CuCl_2$ | $Mg(OAc)_2$ | $MgCl_2$ | r (DMC) | r ($CO_2$) | |
| Example 4 | 0.17 | 19 | | 12.7 | 6.3 | 0.154 | 0.046 | 77.2 |
| Example 5 | 0.17 | 4.7 | 14.3 | 19 | | 0.255 | 0.067 | 79.3 |
| Example 6 | 0.17 | 6.3 | 12.7 | 19 | | 0.202 | 0.057 | 78.0 |
| Example 7 | 0.17 | 9.5 | 9.5 | 19 | | 0.249 | 0.080 | 75.8 |
| Example 8 | 0.17 | 12.7 | 6.3 | 19 | | 0.184 | 0.052 | 77.8 |
| Example 9 | 0.17 | 19 | | | 19 | 0.143 | 0.089 | 61.7 |

Selectivity toward DMC:

$$S(CO_2-DMC) = \frac{r(DMC)}{r(DMC) + r(CO_2)} \times 100$$

It is apparent from the results shown in Table 3 that the DMC-forming activities observed in Examples 4 to 9 were higher than those observed in Comparative Examples 1 and 2 shown in Table 1. Further it will be understood that when the catalyst having an atomic ratio of the halogen to copper in the range of 1/2 to 2 was used, the selectivity toward DMC was remarkably increased in addition to the DMC-forming activity.

Example 10

DMC was produced in a filtration system in a 380-mℓ autoclave coated with Teflon. A solution of 0.34 mmol/ℓ of palladium chloride, 38 mmol/ℓ of copper acetate and 19 mmol/ℓ of magnesium chloride in methanol containing 10 wt. % DMC was used as the catalyst. The reaction temperature was 130°C. 5 vol. % of carbon monoxide, 2.5 vol. % of oxygen and carbon dioxide as the diluent were used. The total pressure was 20 kg/cm². The DMC-forming activity and the selectivity toward DMC were determined according to gas chromatography after the stationary state was realized. The results are shown in Table 4.

Example 11

The reaction was conducted in the same manner as that of Example 10 except that 0.34 mmol/ℓ of palladium chloride, 19 mmol/ℓ of copper acetate, 19 mmol/ℓ of cupric chloride and 38 mmol/ℓ of magnesium acetate were used as the catalyst components. The products were determined according to gas chromatography. The results are shown in Table 4.

Example 12

The reaction was conducted in the same manner as that of Example 10 except that 0.34 mmol/ℓ of palladium chloride, 38 mmol/ℓ of cupric acetate and 38 mmol/ℓ of magnesium chloride were used as the catalyst components. The products were determined according to gas chromatography. The results are shown in Table 4.

Table 4

| | Catalyst composition (mmol/$\ell$) | | | | | Activity (mmol/$\ell \cdot$h) | Selectivity (%) |
| | PdCl$_2$ | Cu(IIAc)$_2$ | CuCl$_2$ | Mg(IIAc)$_2$ | MgCl$_2$ | r(DMC) | S(CO$_2$-DMC) |
|---|---|---|---|---|---|---|---|
| Example 10 | 0.34 | 38 | | | 19 | 1.177 | 43.4 |
| Example 11 | 0.34 | 18 | 19 | 38 | | 1.211 | 47.1 |
| Example 12 | 0.34 | 38 | | | 38 | 0.742 | 49.4 |

11

Selectivity toward DMC:

$$S(CO-DMC) = \frac{\text{Amount of formed DMC (mol)}}{\text{Amount of consumed CO (mol)}} \times 100$$

It is apparent from the results shown in Table 4 that the DMC-forming activity can be remarkably improved when the atomic ratio of the halogen to copper is reduced by merely reducing the amount of magnesium chloride in the catalyst. It is apparent also that the DMC-forming activity can be remarkably increased when the atomic ratio of the halogen to copper is reduced by using palladium chloride, cupric acetate, cupric chloride and magnesium acetate. Namely, the DMC-forming activity can be remarkably improved when the catalyst having the atomic ratio of halogen to copper of higher than 1/2 and lower than 2 is used.

It will be apparent from the above description that the atomic ratio of the halogen to copper in the catalyst is particularly preferably higher than 1/2 and lower than 2.

**Claims**

1.  A process for producing a carbonic acid ester by reacting an alcohol with carbon monoxide and oxygen in the presence of a catalyst homogeneously dissolved in the reaction solution, said catalyst comprising a cupric salt and an alkaline earth metal salt, the cupric salt being a weak acid salt and/or a halide and the alkaline earth metal salt being a weak acid salt and/or a halide.

2.  The process according to claim 1, wherein the catalyst comprises a cupric salt of a weak acid and an alkaline earth metal halide.

3.  The process according to claim 1 or 2 wherein the catalyst further comprises a platinum group compound.

4.  The process according to claim 3, wherein the platinum group compound is a palladium compound, the cupric salt is cupric acetate and/or a cupric halide and the alkaline earth metal salt is an alkaline earth metal acetate and/or halide.

**Patentansprüche**

1.  Verfahren zum Herstellen eines Kohlensäureesters durch Reagierenlassen eines Alkohols mit Kohlenmonoxid und Sauerstoff in Anwesenheit eines in der Reaktionslösung homogen aufgelösten Katalysators, wobei der Katalysator ein Kupfer(II)-Salz und ein Erdalkalimetallsalz umfaßt, wobei das Kupfer(II)-Salz ein schwaches saures Salz und/oder ein Halogenid ist, und das Erdalkalimetallsalz ein schwaches saures Salz und/oder ein Halogenid ist.

2.  Verfahren nach Anspruch 1, wobei der Katalysator ein Kupfer(II)-Salz einer schwachen Säure und ein Erdalkalimetallhalogenid umfaßt.

3.  Verfahren nach Anspruch 1 oder 2, wobei der Katalysator weiterhin eine Verbindung der Platinreihe umfaßt.

4.  Verfahren nach Anspruch 3, wobei die Verbindung der Platinreihe eine Palladiumverbindung ist, das Kupfer(II)-Salz Kupferacetat und/oder Kupferhalogenid ist, und das Erdalkalimetallsalz ein Erdalkalimetallacetat und/oder -halogenid ist.

**Revendications**

1.  Procédé de production d'un ester d'acide carbonique en faisant réagir un alcool avec du monoxyde de carbone et de l'oxygène en présence d'un catalyseur dissous de manière homogène dans la solution réactionnelle, ce catalyseur comprenant un sel cuivrique et un sel dérivé de métal alcalino-terreux, le sel cuivrique étant un sel d'acide faible et/ou un halogénure et le sel dérivé de métal alcalino-terreux étant un acide faible et/ou un halogénure.

2. Procédé selon la revendication 1, dans lequel le catalyseur comprend un sel cuivrique d'un acide faible et un halogénure de métal alcalino-terreux.

3. Procédé selon la revendication 1 ou 2, dans lequel le catalyseur comprend en outre un composé d'un élément du groupe du platine.

4. Procédé selon la revendication 3, dans lequel le composé d'un élément du groupe du platine est un composé du palladium, le sel cuivrique est de l'acétate cuivrique et/ou un halogénure cuivrique, et le sel dérivé de métal alcalino-terreux est un acétate et/ou un halogénure de métal alcalino-terreux.